(19) Europäisches Patentamt
European Patent Office
Office européen des brevets

(11) Veröffentlichungsnummer: **0 393 668 B1**

(12) # EUROPÄISCHE PATENTSCHRIFT

(45) Veröffentlichungstag der Patentschrift: **02.02.94**

(51) Int. Cl.5: **C25B 3/02**, C07C 43/215, C07C 69/025

(21) Anmeldenummer: **90107437.7**

(22) Anmeldetag: **19.04.90**

(54) **Verfahren zur Herstellung von Benzaldehyddialkylacetalen und neue Benzaldehyddialkylacetale.**

(30) Priorität: **21.04.89 DE 3913166**

(43) Veröffentlichungstag der Anmeldung:
**24.10.90 Patentblatt 90/43**

(45) Bekanntmachung des Hinweises auf die Patenterteilung:
**02.02.94 Patentblatt 94/05**

(84) Benannte Vertragsstaaten:
**BE CH DE FR GB IT LI NL**

(56) Entgegenhaltungen:
**EP-A- 0 152 801**

**Organic Electrochemistry, 3rd Ed., Marcel Dekker Inc. 1991, S.635f; S.1082**

(73) Patentinhaber: **BASF Aktiengesellschaft**
**Carl-Bosch-Strasse 38**
**D-67063 Ludwigshafen(DE)**

(72) Erfinder: **Hermeling, Dieter, Dr.**
**Zum Ordenswald 73f**
**D-6730 Neustadt(DE)**
Erfinder: **Harreus, Albrecht, Dr.**
**Teichgasse 13**
**D-6700 Ludwigshafen(DE)**
Erfinder: **Wild, Jochen, Dr.**
**An der Marlach 7**
**D-6705 Deidesheim(DE)**
Erfinder: **Goetz, Norbert, Dr.**
**Schoefferstrasse 25**
**D-6520 Worms 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**Beschreibung**

Diese Erfindung betrifft ein Verfahren zur Herstellung von Benzaldehyddialkylacetalendurch elektrochemische Oxidation von Benzylderivaten sowie neue halogenhaltige Benzaldehyddialkylacetale. Benzaldehyddialkylacetale finden z.B. als lagerstabile Depotverbindungen für die entsprechenden Benzaldehyde Verwendung.

Aus der EP-A-152 801 ist ein Verfahren zur elektrochemischen Oxidation von Benzylalkylethern bekannt.

Die Elektrooxidation von Toluolen zu Benzaldehyddialkylacetalen wird z. B. in der EP-PS 12 240, oder DE-OS 2 848 397 und der DE-OS 2 935 398 beschrieben. Die Benzaldehyddialkylacetale werden dabei allerdings nur dann in guten Ausbeuten erhalten, wenn die Toluole in para Stellung zur Methylgruppe elektronenreiche Substituenten enthalten, die das Oxidationspotential der Toluole absenken. Toluole mit elektronenarmen Substituenten in para Stellung zur Methylgruppe oder beliebigen Substituenten in ortho oder meta Position lassen sich entweder gar nicht oder nur in schlechten Ausbeuten direkt zu Benzaldehyddialkylacetalen elektrooxidieren.

Aus der US-PS 3,448,021 und J. Org. Chem. 51, 4544 (1986) ist bekannt, daß Toluole, die durch ein Halogenatom desaktiviert sind, elektrochemisch zu Benzylacetaten oxidiert werden können.

Halogenierte Benzaldehyde sind wichtige Zwischenprodukte, z.B. für die Herstellung von Wirkstoffen. So werden z.B. 2,4-Dihalogenbenzaldehyde für die Herstellung N-substituierter Tetrahydrophthalimide benötigt, die als Herbizide Verwendung finden (WO 87/4049). Man stellt solche Benzaldehyde nach der gebräuchlichsten Methode durch Halogenierung der entsprechenden Toluole und anschließende Hydrolyse der Dihalogenmethylbenzole her (s. Houben-Weyl, "Methoden der organischen Chemie", 4. Auflage, Band 5/3, Seiten 735-738 und Band 7/1, Seiten 211-214). Von erheblichem Nachteil ist hierbei, daß Dihalogenmethylbenzole carcinogenes Potential besitzen und daß bei der Hydrolyse große Salzmengen anfallen. Wegen dieser Nachteile hat man schon vorgeschlagen, Toluole elektrochemisch zu oxidieren. So lassen sich isomerenreine Benzaldehyde nach den Angaben der Patentschriften DE 2 855 508, EP 72 914 und EP 29 995 durch eine anodische Oxidation aus den entsprechend substituierten Toluolen herstellen. Da auf die Verwendung umweltbelastender Reagenzien verzichtet werden kann, ist die Elektrooxidation anderen chemischen Methoden überlegen. Allerdings ergeben sich bei der Elektrooxidation der Toluole die obengenannten Schwierigkeiten.

Phthalaldehydacetale lassen sich z. B. durch Umsetzung von Bis-(chlormethyl)-benzolen mit Hexamethylentetramin (J. Chem. Soc. 1950, 214) und anschließende Acetalisierung mit o-Estern (J. Chem. Soc., Perkin II, 1975, 1656) oder durch anodische Oxidation von Alkoxymethylbenzolen (DE-OS 34 21 976) herstellen. Die Alkoxymethylbenzole erhält man wiederum aus den entsprechenden Bis-(halogenmethyl)-benzolen (J. Chem. Soc. 1954, 2819). Die Herstellung der seitenkettenhalogenierten Zwischenstufen liefert jedoch nur mäßige Ausbeuten. Da bei der Hydrolyse zudem große Salzmengen gebildet werden und die halogenierten Zwischenstufen gleichzeitig carcinogenes Potential besitzen, sind diese Synthesen wenig umweltfreundlich.

Es wurde nun gefunden, daß man Benzaldehyddialkylacetale der allgemeinen Formel I

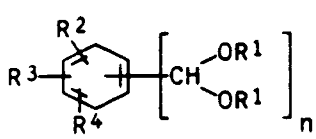

I,

in der $R^1$ einen Alkylrest mit 1 bis 6 C-Atomen, $R^2$, $R^3$ und $R^4$ Wasserstoff-oder Halogenatome, geradkettige, verzweigte oder ringförmige Kohlenwasserstoffreste oder Alkoxy-, Acyloxy-, Aryloxy- oder Aralkoxy-Reste und n die Zahl 1 oder 2 bedeuten, besonders vorteilhaft erhält, wenn man Benzylderivate der allgemeinen Formel II

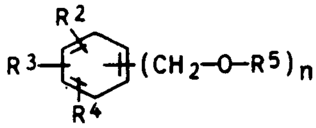

II,

in der $R^2$, $R^3$, $R^4$ und n die obengenannte Bedeutung haben, $R^5$ ein Wasserstoffatom oder einen $R^6$-CO-Rest und $R^6$ ein Wasserstoffatom oder einen Alkylrest mit 1 bis 6 C-Atomen bedeuten, in Gegenwart eines Alkohols der Formel $R^1$-OH, in der $R^1$ die obengenannte Bedeutung hat, anodisch oxidiert.

Der besondere Vorteil des erfindungsgemäßen Verfahrens besteht darin, daß man isomerenreine Benzaldehyddialkylacetale erhält.

Die in den Benzylderivaten der Formel II als Substituenten $R^2$, $R^3$ und $R^4$ in Betracht kommenden geradkettigen, verzweigten oder ringförmigen Kohlenwasserstoffreste haben z.B. 1 bis 18, vorzugsweise 1 bis 12 C-Atome. Kohlenwasserstoffreste dieser Art sind insbesondere geradkettige und verzweigte Alkylreste, Cycloalkylreste oder Arylreste. Als geradkettige Alkylreste seien z. B. Methyl, Ethyl, Propyl, Butyl, Hexyl genannt. Verzweigte Alkylreste sind z. B. solche der Formel

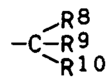 ,

die insgesamt mindestens 3 C-Atome enthalten und wobei die Reste $R^8$, $R^9$ und $R^{10}$ Alkylreste mit 1 bis 6, vorzugsweise 1 bis 3 C-Atomen bedeuten und $R^{10}$ auch für ein Wasserstoffatom stehen kann. Als Reste dieser Art seien z. B. Isopropyl, tert.-Butyl sek.-Butyl und iso-Butyl genannt. Ringförmige Kohlenwasserstoffreste sind solche, die eine Cycloalkylgruppe, eine Bicycloalkylgruppe oder einen Benzol- oder Naphthalinring aufweisen. Die Cycloalkylgruppe enthalten z. B. 3 bis 8 cyclische C-Atome, die ihrerseits Alkylgruppen mit 1 bis 5, vorzugsweise 1 bis 3 C-Atomen tragen können. Cycloalkylreste dieser Art sind z. B. Cyclopropyl, Cyclopentyl, Cyclohexyl, 3,5-Diethylcyclohexyl oder Tetramethylcyclopropyl. Die Bicycloalkylgruppen enthalten z. B. 5 bis 12 bicyclische C-Atome, die ihrerseits Alkylgruppen mit 1 bis 5, vorzugsweise 1 bis 3 C-Atome enthalten können. Als Bicycloalkylgruppen sind z. B. im einzelnen genannt: 2-Norbornyl, Bicyclo[4.1.0]hept-1-yl oder 2,6-Dimethyl-bicyclo[4.1.0]hept-1-yl. Arylgruppen sind z.B. Phenyl, 2-Chlorphenyl oder Biphenyl. Die als weitere Substituenten genannten Alkoxy-, Acyloxy-, Aryloxy- und Aralkoxy-Reste sind z.B. Methoxy, Ethoxy, Acetoxy, Benzoyloxy, Phenoxy, Chlorphenoxy oder tert.-Butylphenoxy. Als Halogenatome sind Fluor-, Chlor- oder Bromatome bevorzugt.

Die als Ausgangsstoffe für das erfindungsgemäße Verfahren verwendeten Benzylester der Formel II, die zum Teil neu sind, lassen sich dadurch gewinnen, daß man die entsprechenden Toluole der Formel IV

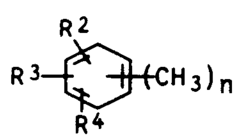 IV,

in Gegenwart einer Alkansäure der Formel $R^6COOH$, in der $R^6$ die obengenannte Bedeutung hat, elektrochemisch oxidiert.

Man kann die Benzaldehyddialkylacetale der Formel I somit besonders vorteilhaft aus den leicht zugänglichen Toluolen der Formel IV, die sich einstufig nicht oder nur in schlechten Ausbeuten elektrochemisch alkoxilieren lassen, in zwei aufeinander folgenden Schritten der elektrochemischen Oxidation herstellen. Dabei ergibt sich der weitere Vorteil, daß der Elektrolyseaustrag der Acyloxilierung nach Abtrennung von Lösungsmittel und Hilfselektrolyt ohne weitere Reinigung direkt für die anodische Alkoxilierung eingesetzt werden kann.

Die beiden elektrochemischen Oxidationen, d.h. das erfindungsgemäße Verfahren zur Herstellung der Benzaldehyddialkylacetale der Formel I, wie auch das Verfahren zur Herstellung der Benzylester der Formel III aus den Toluolen der Formel IV können in an sich üblichen Elektrolysezellen durchgeführt werden. Bevorzugt werden ungeteilte Durchflußzellen eingesetzt. Als Anodenmaterialien dienen z. B. Edelmetalle wie Platin oder Oxide wie $RuO_2$, $Cr_2O_3$ oder $TiO_x/RuO_x$. Bevorzugt werden Anoden aus Graphit eingesetzt. Als Kathodenmaterialien kommen u. a. Eisen, Stahl, Nickel, Edelmetalle, wie Platin oder auch Graphit in Betracht.

Für die anodische Alkoxilierung werden Elektrolyte eingesetzt, die das Benzylderivat der Formel II, den Alkohol der Formel $R^1OH$ und, zur Erhöhung der Leitfähigkeit, einen Hilfselektrolyten enthalten. Als Hilfselektrolyt können die in der Elektrochemie üblichen Leitsalze, wie Fluoride, z.B. KF, Tetrafluorborate, z.B. Natriumtetrafluoroborat, Alkoholate, z.B. Natriummethylat, Sulfonate, z.B. Natriumbenzolsulfonat oder Alkylsulfate, z.B. Natriummethylsulfat verwendet werden.

Der Elektrolyt hat beispielsweise folgende Zusammensetzung:
1 bis 49, vorzugsweise 5 bis 30 Gew.% Benzylverbindung der Formel II,
50 bis 99, vorzugsweise 70 bis 95 Gew.% Alkohol der Formel $R^1OH$
0,1 bis 5, vorzugsweise 0,25 bis 2 Gew.% Hilfselektrolyt.

Man elektrolysiert zweckmäßigerweise bei Stromdichten zwischen 0,2 und 25 A/dm$^2$ und bei Temperaturen bis etwa 100 °C, zweckmäßigerweise zwischen 0 °C und etwa 5 °C unterhalb des Siedepunktes des Alkohols. Die eingesetzten Benzylester bzw. Benzylalkohole lassen sich weitgehend umsetzen. Die Aufarbeitung der Elektrolyseausträge erfolgt nach konventionellen Methoden, vorzugsweise wird destillativ aufgearbeitet. Überschüssiger Alkohol, Leitsalz und ggf. nicht umgesetztes Benzylderivat der Formel II können zur Elektrolyse zurückgeführt werden.

Für die Acyloxilierung verwendet man als Elektrolyten eine Lösung des Toluolderivates der Formel IV in einer Alkansäure der Formel $R^6COOH$, der zur Erhöhung der Leitfähigkeit ein Hilfselektrolyt zugegeben wird. Zur Erhöhung der Löslichkeit der Toluole kann man auch ein Colösungsmittel zugeben. Geeignete Colösungsmittel sind z.B. Ketone, wie Aceton oder Methylethylketon, Nitrile, wie Acetonitril oder Propionitril und Anhydride, wie Acetanhydrid. Als Hilfselektrolyte werden z.B. die oben genannten Leitsalze verwendet. Ein für die elektrochemische Acyloxilierung geeigneter Elektrolyt hat z.B. die folgende Zusammensetzung:
1 bis 40 Gew.% Toluol der allgemeinen Formel IV, 1 bis 10 Gew.% Leitsalz, 0 bis 20 Gew.% Colösungsmittel und 50 bis 95 Gew.% Alkansäure.

Die Stromdichten und Elektrolysetemperaturen lassen sich in weiten Grenzen variieren. So wird z.B. bei 0,2 bis 25 A/dm$^2$ und bei 15 bis 95 °C elektrolysiert. Die eingesetzten Toluole lassen sich weitgehend umsetzen, die Aufarbeitung der Elektrolyseausträge erfolgt nach an sich bekannten Methoden, z.B. durch Destillation, Extraktion und Kristallisation. Colösungsmittel, überschüssige Alkansäure und Leitsalz können nach Abtrennung von den Benzylestern zusammen mit gegebenenfalls nicht umgesetztem Toluol zur Elektrolyse zurückgeführt werden.

Als Alkohole der Formel $R^1OH$ kommen z.B. Methanol, Ethanol, Propanol und Butanol in Betracht. Als Carbonsäuren der Formel $R^6COOH$ seien z.B. Ameisensäure, Essigsäure oder Propionsäure genannt.

Diese Erfindung betrifft ferner die neuen halogenhaltigen Benzaldehyddialkylacetale der allgemeinen Formeln

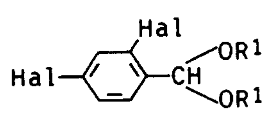

und

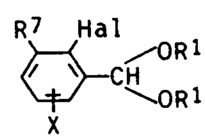

V

VI

in denen Hal ein Halogenatom, $R^1$ einen Alkylrest mit 1 bis 6, vorzugsweise 1 bis 4 C-Atomen, $R^7$ einen geradkettigen, verzweigten oder ringförmigen Kohlenwasserstoffrest mit 1 bis 18 C-Atomen, X ein Wasserstoff- oder Halogenatom und $R^6$ ein Wasserstoffatom oder einen Alkylrest mit 1 bis 6, vorzugsweise 1 bis 4 C-Atomen bedeuten. Von den neuen Benzaldehyddialkylacetalen der Formel VI sind solche von besonderem technischen Interesse, in denen der Rest $R^7$ für einen verzweigten oder ringförmigen Alkylrest mit 3 bis 12, insbesondere 3 bis 8 C-Atomen steht.

Die neuen Benzaldehyddialkylacetale der Formeln V und VI lassen sich leicht zu den entsprechenden Benzaldehyden verseifen. Sie stellen somit lagerstabile Depotverbindungen für Benzaldehyde der Formeln

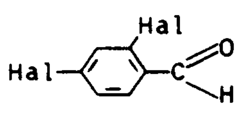

und

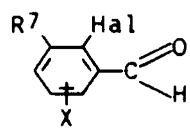

VIII

IX

dar. Die Verseifung der Diacetale nimmt man z.B. nach an sich bekannten Methoden vor, beispielsweise durch einfaches Erhitzen in Wasser unter Zusatz katalytischer Mengen Säure auf Temperaturen von 40 bis 95 °C.

Als 2,4-Dihalogentoluole, die man zur Herstellung von Benzylestern der Formel III und der Benzaldehyddialkylacetale der Formel V heranzieht, seien z.B. 2,4-Dichlortoluol, 2,4-Difluortoluol, 2-Chlor-4-fluortoluol,

2,4-Dibromtoluol, 2-Fluor-4-chlortoluol und 2-Brom-4-fluortoluol genannt.

Als Benzylderivate der Formel II, die man für die Herstellung der neuen Benzaldehyddialkylacetale der Formel VI verwendet, kommen z.B. die aus der folgenden Tabelle ersichtlichen Verbindungen der Formel

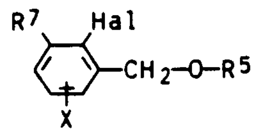

in Betracht.

| Nr. | Hal | $R^7$ | $R^5$ | X |
|---|---|---|---|---|
| 1 | Cl | 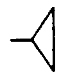 | $CH_3-CO-$ | H |
| 2 | Cl | $-CH(CH_3)-CH_2-CH_3$ | $CH_3-CO-$ | H |
| 3 | Br | $-CH(CH_3)_2$ | H | H |
| 4 | Br | $-CH(CH_3)_2$ | $CH_3-CO-$ | H |
| 5 | Cl | 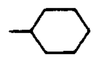 | $CH_3-CO-$ | H |
| 6 | Cl | 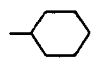 | H | H |
| 7 | F | $-CH(CH_3)_2$ | H | H |
| 8 | F | $-CH(CH_3)_2$ | $CH_3-CO-$ | H |
| 9 | Cl | 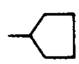 | H | H |
| 10 | Cl | 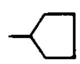 | $CH_3-CO-$ | H |
| 11 | F | 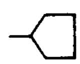 | H | 6-Cl |
| 12 | F | 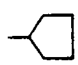 | $CH_3-CO-$ | 6-Cl |
| 13 | Cl | $-CH(CH_3)_2$ | $CH_3-CO-$ | H |
| 14 | Cl | $-CH(CH_3)_2$ | H | H |
| 15 | Cl | $-CH(CH_3)_2$ | $CH_3-CO-$ | 6-F |
| 16 | Cl | $-CH(CH_3)_2$ | H | 6-F |
| 17 | Cl | $-C(CH_3)_3$ | $CH_3-CO-$ | H |
| 18 | Cl | $-C(CH_3)_3$ | H | H |
| 19 | Cl | $-CH(CH_3)-CH_2-CH_3$ | H | H |
| 20 | F | 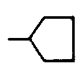 | $CH_3-CO-$ | H |
| 21 | F | 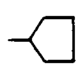 | H | H |
| 22 | Cl | $-CH(CH_3)_2$ | $H-CO-$ | H |
| 23 | Cl | $-CH(CH_3)_2$ | $CH_3-CH_2-CO-$ | H |
| 24 | F | $-C(CH_3)_3$ | $CH_3-CO-$ | H |
| 25 | F | $-C(CH_3)_3$ | H | H |

Beispiel 1

2-Methylbenzaldehyddimethylacetal

a) Elektrosynthese von 2-Methylbenzylacetat
o-Xylol wird in der wie folgt beschriebenen Elektrolysezelle unter den angegebenen Bedingungen elektrolysiert.
Apparatur: ungeteilte Zelle mit 11 bipolaren Elektroden
Anode: Graphit
Elektrolyt: 1350 g (12,74 Mol) o-Xylol, 270 g Acetanhydrid, 270 g Triethylmethylammoniummethylsulfat und 7110 g Essigsäure
Kathode: Graphit
Stromdichte: 1,33 $A/dm^2$
Elektrolysetemperatur: 75 °C
Elektrolyse mit 2,75 F/mol o-Xylol. Der Elektrolyt wird während der Elektrolyse mit 200 l/h durch die Zelle gepumpt. Nach Beendigung der Elektrolyse werden Essigsäure/Acetanhydrid bei Normaldruck bis 150 °C abdestilliert und der Rückstand zwischen Wasser/Methyl-tert.butylether verteilt. Aus der organischen Phase wird Methyl-tert.butylether bei Normaldruck abdestilliert. Man erhält 1930 g Rückstand, der nach gaschromatographischer Analyse 79,4 g (5 %) 2-Methylbenzaldehyd und 1222 g (59 %) 2-Methylbenzylacetat enthält. 2-Methylbenzylacetat wird bei 2 mbar und 72 °C reindestilliert.
b) Elektrosynthese von 2-Methylbenzaldehyddimethylacetal
2-Methylbenzylacetat wird in der in Absatz (a) beschriebenen Elektrolysezelle unter den folgenden Bedingungen elektrooxidiert:
Elektrolyt: 375 g (2,287 Mol) 2-Methylbenzylacetat, 18,75 g Natriumbenzolsulfonat, 6,25 g Natriummethylat und 2100 g Methanol Stromdichte: 3,4 $A/dm^2$
Elektrolysetemperatur: 25 °C
Elektrolyse mit 3,75 F/mol 2-Methylbenzylacetat. Der Elektrolyt wird während der Elektrolyse mit 200 l/h durch die Zelle gepumpt. Nach Beendigung der Elektrolyse wird das Methanol bei Normaldruck abdestilliert, das ausgefallene Leitsalz abfiltriert und das Filtrat im Vakuum reindestilliert. Man erhält 14,6 g (5 %) 2-Methylbenzaldehyd und 248,5 g (66 %) 2-Methylbenzaldehyddimethylacetal (Sdp.: 46 - 48 °C/2 mbar).

Beispiel 2

3-Methylbenzaldehyddimethylacetal

a) Elektrosynthese von 3-Methylbenzylacetat
m-Xylol wird in der in Beispiel 1a beschriebenen Elektrolysezelle unter den folgenden Bedingungen elektrolysiert:
Elektrolyt: 900 g (8,49 Mol) m-Xylol, 180 g Triethylmethylammoniummethylsulfat, 180 g Acetanhydrid und 4740 g Essigsäure
Stromdichte: 1 $A/dm^2$
Elektrolysetemperatur: 77 °C
Elektrolyse mit 3,25 F/mol m-Xylol. Der Elektrolyt wird während der Elektrolyse mit 200 l/h durch die Zelle gepumpt. Die Aufarbeitung erfolgt wie in Beispiel 1a) beschrieben. Nach Reindestillation im Vakuum erhält man 566,4 g (41 %) 3-Methylbenzylacetat (Sdp.: 75 °C/2 mbar).
b) Elektrosynthese von 3-Methylbenzaldehyddimethylacetal
3-Methylbenzylacetat wird in der in Beispiel 1a beschriebenen Elektrolysezelle unter den folgenden Bedingungen elektrooxidiert:
Elektrolyt: 375 g (2,29 Mol) 3-Methylbenzylacetat, 18,75 g Natriumbenzolsulfonat, 6,25 g Natriummethylat und 2100 g Methanol
Stromdichte: 3,4 $A/dm^2$
Elektrolysetemperatur: 25 °C
Elektrolyse mit 3,5 F/mol 3-Methylbenzylacetat. Der Elektrolyt wird während der Elektrolyse mit 200 l/h durch die Zelle gepumpt. Die Aufarbeitung des Elektrolyseaustrags erfolgt wie in Beispiel 1b) beschrieben. Nach Reindestillation im Vakuum erhält man 14,9 g (5 %) 3-Methylbenzaldehyd und 229,3 g (60 %) 3-Methylbenzaldehyddimethylacetal (Sdp.: 62 °C/3 mbar).

Beispiel 3

3,5-Dimethylbenzaldehyddimethylacetal

a) Elektrosynthese von 3,5-Dimethylbenzylacetat
Mesitylen wird in der in Beispiel 1a beschriebenen Elektrolysezelle unter den folgenden Bedingungen elektrolysiert:
Elektrolyt: 945 g (7,875 Mol) Mesitylen, 210 g Kaliumbenzolsulfonat, 945 g Acetanhydrid und 5300 g Essigsäure
Stromdichte: 0,5 $A/dm^2$
Elektrolysetemperatur: 70 °C
Elektrolyse mit 2,5 F/mol Mesitylen. Der Elektrolyt wird während der Elektrolyse mit 200 l/h durch die Zelle gepumpt. Die Aufarbeitung des Elektrolyseaustrags erfolgt wie in Beispiel 1a) beschrieben. Nach Reindestillation im Vakuum erhält man 698,1 g (50 %) 3,5-Dimethylbenzylacetat (Sdp.: 96 °C/1 mbar).
b) Elektrosynthese von 3,5-Dimethylbenzaldehyddimethylacetal
3,5-Dimethylbenzylacetat wird in der in Beispiel 1a beschriebenen Elektrolysezelle unter den folgenden Bedingungen elektrooxidiert:
Elektrolyt: 665 g (3,736 Mol) 3,5-Dimethylbenzylacetat, 30 g Natriumbenzolsulfonat, 30 g Natriummethylat und 5290 g Methanol
Stromdichte: 3,4 $A/dm^2$
Elektrolysetemperatur: 40 °C
Elektrolyse mit 5 F/mol 3,5-Dimethylbenzylacetat. Der Elektrolyt wird während der Elektrolyse mit 200 l/h durch die Zelle gepumpt. Die Aufarbeitung des Elektrolyseaustrags erfolgt wie in Beispiel 1b) beschrieben. Bei einem Oxidationsgrad von 89 % erhält man nach Reindestillation im Vakuum 18,1 g (4 %) 3,5-Dimethylbenzaldehyd und 292,4 g (44 %) 3,5-Dimethylbenzaldehyddimethylacetal (Sdp. 67 °C/2 mbar). Hieraus errechnet sich eine Selektivität von 54 %.

Beispiel 4

4-Fluorbenzaldehyddimethylacetal

a) Elektrosynthese von 4-Fluorbenzylacetat
4-Fluortoluol wird in der in Beispiel 1a beschriebenen Elektrolysezelle unter den folgenden Bedingungen elektrolysiert:
Elektrolyt: 190 g (1,727 Mol) 4-Fluortoluol, 190 g Acetanhydrid, 57 g Triethylmethylammoniummethylsulfat und 1663 g Essigsäure
Stromdichte: 1,33 $A/dm^2$
Elektrolysetemperatur: 70 °C
Elektrolyse mit 3,75 F/mol 4-Fluortoluol. Der Elektrolyt wird während der Elektrolyse mit 200 l/h durch die Zelle gepumpt. Die Aufarbeitung des Elektrolyseaustrags erfolgt wie in Beispiel 1a) beschrieben. Man erhält 232 g Rückstand, der nach gaschromatographischer Analyse 43 g (23 %) 4-Fluortoluol, 11,3 g (5 %) 4-Fluorbenzaldehyd und 179,3 g (62 %) 4-Fluorbenzylacetat enthält. 4-Fluorbenzylacetat wird bei 2 mbar und 70 °C reindestilliert.
b) Elektrosynthese von 4-Fluorbenzaldehyddimethylacetal
4-Fluorbenzylacetat wird in der in Beispiel 1a beschriebenen Elektrolysezelle unter den folgenden Bedingungen elektrooxidiert:
Elektrolyt: 250 g (1,488 Mol) 4-Fluorbenzylacetat, 12,5 g Natriumbenzolsulfonat, 12,5 g Natriummethylat und 2225 g Methanol
Stromdichte: 3,4 $A/dm^2$
Elektrolysetemperatur: 25 °C
Elektrolyse mit 4 F/mol 4-Fluorbenzylacetat. Der Elektrolyt wird während der Elektrolyse mit 200 l/h durch die Zelle gepumpt. Nach Beendigung der Elektrolyse wird der Austrag mit Natriummethylat auf pH 8 bis 10 eingestellt und anschließend wie in Beispiel 1b) beschrieben, weiter aufgearbeitet. Bei einem Oxidationsgrad von 94 % erhält man nach Reindestillation im Vakuum 7,7 g (4 %) 4-Fluorbenzaldehyd und 135,4 g (54 %) 4-Fluorbenzaldehyddimethylacetal (Sdp.: 42 °C/2 mbar). Hieraus errechnet sich eine Selektivität von 62 %.

Beispiel 5

2-Fluorbenzaldehyddimethylacetal

a) Elektrosynthese von 2-Fluorbenzylacetat
2-Fluortoluol wird in der in Beispiel 1a beschriebenen Elektrolysezelle unter den folgenden Bedingungen elektrooxidiert:
Elektrolyt: 1013 g (9,206 Mol) 2-Fluortoluol, 675 g Acetanhydrid, 338 g Triethylmethylammoniummethylsulfat und 4727 g Essigsäure
Stromdichte: 1,33 $A/dm^2$
Elektrolysetemperatur: 75 °C
Elektrolyse mit 4,5 F/mol 2-Fluortoluol. Der Elektrolyt wird während der Elektrolyse mit 200 l/h durch die Zelle gepumpt. Die Aufarbeitung des Elektrolyseaustrags erfolgt wie in Beispiel 1a) beschrieben. Nach Reindestillation im Vakuum erhält man 835,2 g (54 %) 2-Fluorbenzylacetat (Sdp. 80 °C/4 mbar).
b) Elektrosynthese von 2-Fluorbenzaldehyddimethylacetal
2-Fluorbenzylacetat wird in der in Beispiel 1a beschriebenen Elektrolysezelle unter den folgenden Bedingungen elektrooxidiert:
Elektrolyt: 740 g (4,405 Mol) 2-Fluorbenzylacetat, 50 g Natriumbenzolsulfonat, 25 g Natriummethylat und 4185 g Methanol.
Stromdichte: 3,4 $A/dm^2$
Elektrolysetemperatur: 40 °C
Elektrolyse mit 7 F/mol 2-Fluorbenzylacetat. Der Elektrolyt wird während der Elektrolyse mit 200 l/h durch die Zelle gepumpt. Nach Beendigung der Elektrolyse wird der Austrag mit Natriummethylat auf pH 8 - 10 eingestellt und anschließend wie in Beispiel 1b) beschrieben, weiter aufgearbeitet. Bei einem Oxidationsgrad von 91 % erhält man nach Reindestillation im Vakuum 348,8 g (47 %) 2-Fluorbenzaldehyddimethylacetal (Sdp.: 40 °C/2 mbar). Die Selektivität der anodischen Methoxilierung beträgt somit 52 %.

Beispiel 6

4-(4-Methylphenyl)benzaldehyddimethylacetal

a) Elektrosynthese von 4-(4-Methylphenyl)benzylacetat
4-(4-Methylphenyl)toluol wird in der in Beispiel 1a beschriebenen Elektrolysezelle unter den folgenden Bedingungen elektrooxidiert:
Elektrolyt: 350 g (1,923 Mol) 4-(4-Methylphenyl)toluol, 600 g Acetanhydrid, 186 g Kaliumbenzolsulfonat und 6200 g Essigsäure
Stromdichte: 0,33 $A/dm^2$
Elektrolysetemperatur: 70 °C
Elektrolyse mit 3 F/mol 4-(4-Methylphenyl)toluol. Der Elektrolyt wird während der Elektrolyse mit 200 l/h durch die Zelle gepumpt. Die Aufarbeitung des Elektrolyseaustrags erfolgt wie in Beispiel 1a) beschrieben. Nach Reindestillation im Vakuum erhält man 5,5 g (2 %) 4-(4-Methylphenyl)toluol und 245,1 g (53 %) 4-(4-Methylphenyl)benzylacetat (Sdp. 172 - 178 °C/3 mbar; Schmp.: 46 °C).
b) Elektrosynthese von 4-(4-Methylphenyl)benzaldehyddimethylacetal
4-(4-Methylphenyl)benzylacetat wird in der in Beispiel 1a beschriebenen Elektrolysezelle unter den folgenden Bedingungen elektrooxidiert:
Elektrolyt: 171 g (0,713 Mol) 4-(4-Methylphenyl)benzylacetat, 30 g Natriumbenzolsulfonat, 15 g Natriummethylat und 2784 g Methanol
Stromdichte: 3,4 $A/dm^2$
Elektrolysetemperatur: 40 °C
Elektrolyse mit 6 F/mol 4-(4-Methylphenyl)benzylacetat. Der Elektrolyt wird während der Elektrolyse mit 200 l/h durch die Zelle gepumpt. Die Aufarbeitung des Elektrolyseaustrags erfolgt wie in Beispiel 1b) beschrieben. Bei einem Oxidationsgrad von 96,5 % erhält man nach Reindestillation im Vakuum 5,5 g (4 %) 4-(4-Methylphenyl)benzaldehyd und 63,3 g (37 %) 4-(4-Methylphenyl)benzaldehyddimethylacetal (Sdp. 148 - 155 °C/3 mbar). Hieraus errechnet sich eine Selektivität von 42,5%.

Beispiel 7

2-Chlor-4-fluorbenzaldehyddimethylacetal

a) Elektrosynthese von 2-Chlor-4-fluorbenzylacetat
2-Chlor-4-fluortoluol wird in der in Beispiel 1a beschriebenen Elektrolysezelle unter den folgenden Bedingungen oxidiert:
Elektrolyt: 1200 g (8,307 Mol) 2-Chlor-4-fluortoluol, 400 g Acetanhydrid, 200 g Triethylmethylammoniummethylsulfat und 6200 g Essigsäure
Stromdichte: 1,33 A/dm$^2$
Elektrolysetemperatur: 70 °C
Elektrolyse mit 4,5 F/mol 2-Chlor-4-fluortoluol. Der Elektrolyt wird während der Elektrolyse mit 200 l/h durch die Zelle gepumpt. Die Aufarbeitung des Elektrolyseaustrags erfolgt wie in Beispiel 1a beschrieben. Man erhält 1575 g Rückstand, der nach gaschromatographischer Analyse 10,1 g 2-Chlor-4-fluortoluol, 80,1 g 2-Chlor-4-fluorbenzaldehyd und 1081,2 g 2-Chlor-4-fluorbenzylacetat enthält. Hieraus errechnen sich ein Umsatz von 99,2 %, eine Ausbeute von 64,3 % und eine Selektivität von 71,0 %. 2-Chlor-4-fluorbenzylacetat wird bei 2 mbar und 86-88 °C reindestilliert.
$^1$H-NMR (300 MHz, $CDCl_3$):
(ppm) = 2.12 (s, 3H, $CH_3COO$-); 5.18 (s, 2H, -$CH_2OAc$); 7.0, 7.16, 7.4 (jeweils m, 3H, arom.-H)
b) Herstellung von 2-Chlor-4-fluorbenzaldehyddimethylacetal
2-Chlor-4-fluorbenzylacetat wird in der in Beispiel 1a beschriebenen Elektrolysezelle unter folgenden Bedingungen oxidiert:
Elektrolyt: 900 g (4,445 Mol) 2-Chlor-4-fluorbenzylacetat, 30 g Natriummethylat, 90 g Natriumbenzolsulfonat und 5040 g Methanol
Stromdichte: 3,3 A/dm$^2$
Elektrolysetemperatur: 45-50 °C
Elektrolyse mit 5 F/mol 2-Chlor-4-fluorbenzylacetat. Der Elektrolyt wird während der Elektrolyse mit 200 l/h durch die Zelle gepumpt. Nach Beendigung der Elektrolyse wird der Austrag mit Natriummethylat auf pH 7-8 eingestellt und anschließend wie in Beispiel 1b) beschrieben, weiter aufgearbeitet. Nach Reindestillation im Vakuum erhält man 44,9 g 2-Chlor-4-fluorbenzaldehyd und 675,7 g 2-Chlor-4-fluorbenzaldehyddimethylacetal (Sdp.: 60-62 °C/2 mbar). Hieraus errechnen sich eine Ausbeute von 74,2 % und eine Selektivität von 80,6 %.
$^1$H-NMR (300 MHz, $CDCl_3$):
(ppm) = 3.37 (s, 6H, $OCH_3$); 5.59 (s, 1H, -$CH(OCH_3)_2$); 7.0, 7.13, 7.6 (jeweils m, 3H, arom.-H).

Beispiel 8

Terephthaldialdehydtetramethylacetal

a) Elektrosynthese von p-Xylylendiacetat
p-Xylol wird in der in Beispiel 1a beschriebenen Elektrolysezelle unter den folgenden Bedingungen elektrolysiert:
Elektrolyt: 300 g (2,83 Mol) p-Xylol, 300 g Acetanhydrid, 120 g Triethylmethylammoniummethylsulfat und 2280 g Essigsäure
Stromdichte: 1,33 A/dm$^2$
Elektrolysetemperatur: 75 °C Elektrolyse mit 5 F/mol p-Xylol. Der Elektrolyt wird während der Elektrolyse mit 200 l/h durch die Zelle gepumpt. Die Aufarbeitung des Elektrolyseaustrags erfolgt wie in Beispiel 1a) beschrieben. Nach Reindestillation im Vakuum erhält man 194,1 g (31 %) p-Xylylendiacetat (Sdp.: 125 °C/2 mbar).
b) Elektrosynthese von Terephthaldialdehydtetramethylacetal
p-Xylylendiacetat wird in der in Beispiel 1a beschriebenen Elektrolysezelle unter den folgenden Bedingungen elektrooxidiert:
Elektrolyt: 450 g (2,027 Mol) p-Xylylendiacetat, 15 g Natriummethylat und 2542 g Methanol
Stromdichte: 3,4 A/dm$^2$
Elektrolysetemperatur: 40 °C
Elektrolyse mit 6,5 F/mol p-Xylylendiacetat unter Zusatz von 30 g Schwefelsäure nach 0,5 F/mol. Der Elektrolyt wird während der Elektrolyse mit 200 l/h durch die Zelle gepumpt. Nach Beendigung der Elektrolyse wird der Austrag durch Zugabe von Natriummethylat auf pH 8 bis 9 gestellt und anschließend

wie in Beispiel 1b beschrieben, weiter aufgearbeitet. Nach Reindestillation im Vakuum erhält man 44,8 g (12,3 %) Terephthaldialdehyddimethylacetal und 275,1 g (60,1 %) Terephthaldialdehydtetramethylacetal. Hieraus errechnet sich eine Selektivität von 72,3 %. Terephthaldialdehydtetramethylacetal wird bei 115 °C und 5 mbar reindestilliert.

Beispiel 9

Isophthaldialdehydtetramethylacetal

a) Elektrosynthese von m-Xylylendiacetat
m-Xylol wird in der in Beispiel 1a beschriebenen Elektrolysezelle unter den folgenden Bedingungen elektrolysiert:
Elektrolyt: 450 g (4,245 Mol) m-Xylol, 300 g Acetanhydrid, 150 g Triethylmethylammoniummethylsulfat und 2100 g Essigsäure
Stromdichte: 1,33 A/dm$^2$
Elektrolysetemperatur: 75 °C
Elektrolyse mit 7 F/mol m-Xylol. Der Elektrolyt wird während der Elektrolyse mit 200 l/h durch die Zelle gepumpt. Die Aufarbeitung des Elektrolyseaustrags erfolgt wie in Beispiel 1a) beschrieben. Nach Reindestillation im Vakuum erhält man 225,1 g (24 %) m-Xylylendiacetat (Sdp.: 120 - 125 °C/2 mbar).
b) Elektrosynthese von Isophthaldialdehydtetramethylacetal
m-Xylylendiacetat wird in der in Beispiel 1a beschriebenen Elektrolysezelle unter den folgenden Bedingungen elektrolysiert:
Elektrolyt: 216 g (0,973 Mol) m-Xylylendiacetat, 30 g Natriummethylat, 15 g Natriumbenzolsulfonat und 2755 g Methanol
Stromdichte: 3,4 A/dm$^2$
Elektrolysetemperatur: 40 °C
Elektrolyse mit 10 F/mol m-Xylylendiacetat. Der Elektrolyt wird während der Elektrolyse mit 200 l/h durch die Zelle gepumpt. Die Aufarbeitung des Elektrolyseaustrags erfolgt wie in Beispiel 1b) beschrieben. Bei einem Umsatz von 89,3 % erhält man nach Reindestillation im Vakuum 59,2 g (27,0 %) Isophthaldialdehydtetramethylacetal. Hieraus errechnet sich eine Selektivität von 30,2 %. Isophthaldialdehydtetramethylacetal wird bei 108 °C und 3 mbar reindestilliert.

Beispiel 10

2-Chlor-3-isopropylbenzaldehyddimethylacetal

2-Chlor-3-isopropylbenzylacetat wird in der in Beispiel 1a beschriebenen Elektrolysezelle unter den folgenden Bedingungen oxidiert:
Elektrolyt: 290 g (1,28 Mol) 2-Chlor-3-isopropylbenzylacetat, 30 g Natriumbenzolsulfonat, 15 g Natriummethylat und 2665 g Methanol
Stromdichte: 3,3 A/dm$^2$
Elektrolysetemperatur: 40 °C
Elektrolyse mit 8 F/mol 2-Chlor-3-isopropylbenzylacetat. Der Elektrolyt wird während der Elektrolyse mit 200 l/h durch die Zelle gepumpt. Die Aufarbeitung des Elektrolyseaustrags erfolgt wie in Beispiel 1b) beschrieben. Nach Reindestillation im Vakuum erhält man 166,7 g (57 %) 2-Chlor-3-isopropylbenzaldehyddimethylacetal (Sdp.: 92 °C/2 mbar).
$^1$H-NMR (200 MHz, $CDCl_3$):
$\delta$ (ppm) = 1.28 (d, 6H, $-CH_3$), 3.45 (s, 6H, $-OCH_3$); 3.50 (m, 1H, $-CH(CH_3)_2$); 5.71 (s, 1H, $-CH(OCH_3)_2$); 7.33 (m, 2H, arom.-H); 7.51 (m, 1H, arom.-H).

Beispiel 11

2-Chlor-3-cyclopentylbenzaldehyddimethylacetal

2-Chlor-3-cyclopentylbenzylacetat wird in der in Beispiel 1a beschriebenen Elektrolysezelle unter den folgenden Bedingungen oxidiert:
Elektrolyt: 98 g (0,388 Mol) 2-Chlor-3-cyclopentylbenzylacetat, 30 g Natriumbenzolsulfonat, 15 g Natriummethylat und 2857 g Methanol.

Elektrolyse mit 18 F/mol 2-Chlor-3-cyclopentylbenzylacetat. Der Elektrolyt wird während der Elektrolyse mit 200 l/h durch die Zelle gepumpt. Die Aufarbeitung des Elektrolyseaustrags erfolgt wie in Beispiel 1b) beschrieben. Nach Reindestillation im Vakuum erhält man 40,4 g (41 %) 2-Chlor-3-cyclopentylbenzaldehyddimethylacetal (Sdp.: 140 - 145 °C/4 mbar).

$^1$H-NMR (270 MHz, $CDCl_3$):

δ (ppm) = 1.5 - 2.15 (m, 8H,-$CH_2$), 3.34 (s, 6H, -$OCH_3$); 3.52 (m, 1H, -CH); 5.63 (s, 1H, -$CH(OCH_3)_2$); 7.2 (m, 2H, arom.-H); 7.44 (d, 1H, arom.-H).

$^{13}$C-NMR (270 MHz, $CDCl_3$)

δ (ppm) = 25.6 (t, 2C), 33.4 (t, 2C), 42,4 (d), 53.8 (q, 2C), 101.9 (d), 125.7 (d), 126.3 (d), 127.2 (d), 133.3 (s), 136.1 (s), 144.2 (s).

## Patentansprüche

**1.** Verfahren zur Herstellung von Benzaldehyddialkylacetalen der allgemeinen Formel I

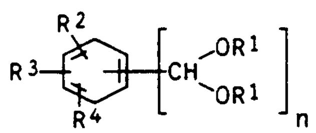 I,

in der $R^1$ einen Alkylrest mit 1 bis 6 C-Atomen, $R^2$, $R^3$ und $R^4$ Wasserstoff-oder Halogenatome, geradkettige, verzweigte oder ringförmige Kohlenwasserstoffreste oder Alkoxy-, Acyloxy-, Aryloxy- oder Aralkoxy-Reste und n die Zahl 1 oder 2 bedeuten, dadurch gekennzeichnet, daß man Benzylderivate der allgemeinen Formel II

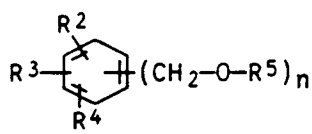 II,

in der $R^2$, $R^3$, $R^4$ und n die obengenannte Bedeutung haben, $R^5$ ein Wasserstoffatom oder einen $R^6$-CO-Rest und $R^6$ ein Wasserstoffatom oder einen Alkylrest mit 1 bis 6 C-Atomen bedeuten, in Gegenwart eines Alkohols der Formel $R^1$-OH, in der $R^1$ die obengenannte Bedeutung hat, anodisch oxidiert.

**2.** Verfahren nach Anspruch 1, dadurch gekennzeichnet, daß man als Benzylderivate der allgemeinen Formel II Benzylester der allgemeinen Formel III

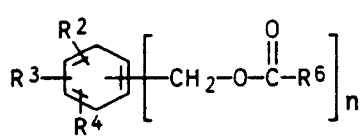 III,

in der $R^2$, $R^3$, $R^4$, $R^6$ und n die in Anspruch 1 genannte Bedeutung haben, verwendet, die durch elektrochemische Oxidation von Toluolderivaten der Formel IV

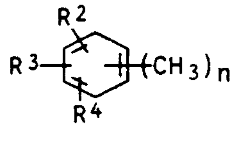 IV,

in der $R^2$, $R^3$, $R^4$ und n die obengenannte Bedeutung haben, in Gegenwart einer Alkansäure der Formel $R^6COOH$, in der $R^6$ die obengenannte Bedeutung hat, erhalten wurden.

**3.** Verfahren nach Anspruch 1, dadurch gekennzeichnet, daß man für die Elektrooxidation einen Elektrolyten mit der Zusammensetzung 1 bis 49 Gew.% Benzylderivat der Formel II, 50 bis 99 Gew.% eines Alkohols der Formel $R^1OH$ und 0,1 bis 5 Gew.% eines Hilfselektrolyten heranzieht.

**4.** Verfahren nach Anspruch 1, dadurch gekennzeichnet, daß man die Elektrooxidation bei Stromdichten von 0,2 bis 25 $A/dm^2$ und bei Temperaturen bis 100 °C durchführt.

**5.** Benzaldehyddialkylacetale der allgemeinen Formel V

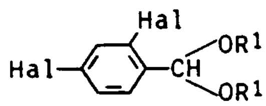

V,

in der Hal ein Halogenatom und $R^1$ einen Alkylrest mit 1 bis 6 C-Atomen bedeuten.

**6.** Benzaldehyddialkylacetale der allgemeinen Formel VI

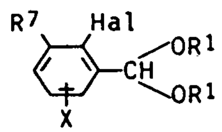

VI,

in der Hal ein Halogenatom, $R^1$ einen Alkylrest mit 1 bis 6 C-Atomen, $R^7$ einen geradkettigen, verzweigten oder ringförmigen Kohlenwasserstoffrest mit 1 bis 18 C-Atomen und X ein Wasserstoff- oder Halogenatom bedeuten.

**7.** Verwendung der Benzaldehyddialkylacetale nach den Ansprüchen 5 und 6 für die Herstellung von Benzaldehyden der Formeln

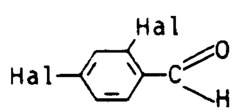

VIII

und

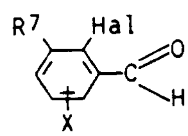

IX

,

in denen Hal, $R^7$ und X die in den Ansprüchen 5 und 6 genannte Bedeutung haben.

## Claims

**1.** A process for the preparation of a benzaldehyde dialkyl acetal of the formula I

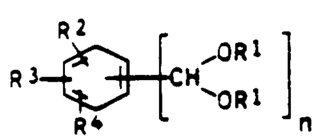

I

where $R^1$ is alkyl of 1 to 6 carbon atoms, $R^2$, $R^3$ and $R^4$ are hydrogen or halogen, straight-chain, branched or cyclic hydrocarbon radicals or alkoxy, acyloxy, aryloxy or aralkoxy, and n is 1 or 2, which comprises anodic oxidation of a benzyl derivative of the formula II

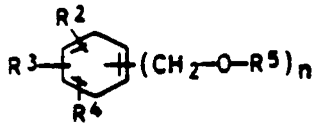

II

where $R^2$, $R^3$, $R^4$ and n have the abovementioned meanings, $R^5$ is hydrogen or $R^6$-CO-, and $R^6$ is hydrogen or alkyl of 1 to 6 carbon atoms, in the presence of an alcohol of the formula $R^1$-OH where $R^1$ has the abovementioned meaning.

**2.** A process as claimed in claim 1, in which the benzyl derivative of the formula II which is used is a benzyl ester of the formula III

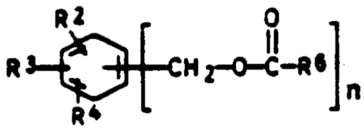

III

where $R^2$, $R^3$, $R^4$, $R^6$ and n have the meanings specified in claim 1, which has been obtained by electrochemical oxidation of a toluene derivative of the formula IV

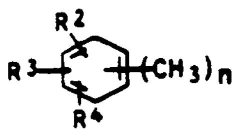

IV

where $R^2$, $R^3$, $R^4$ and n have the abovementioned meanings, in the presence of an alkanoic acid of the formula $R^6COOH$ where $R^6$ has the abovementioned meaning.

**3.** A process as claimed in claim 1, wherein an electrolyte with the composition 1 to 49% by weight of benzyl derivative of the formula II, 50 to 99% by weight of an alcohol of the formula $R^1OH$ and 0.1 to 5% by weight of an auxiliary electrolyte is used for the electrochemical oxidation.

**4.** A process as claimed in claim 1, wherein the electrochemical oxidation is carried out at current densities of from 0.2 to 25 A/dm$^2$ and at up to 100 ° C.

**5.** A benzaldehyde dialkyl acetal of the formula V

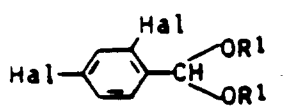

V

where Hal is halogen and $R^1$ is alkyl of 1 to 6 carbon atoms.

**6.** A benzaldehyde dialkyl acetal of the formula VI

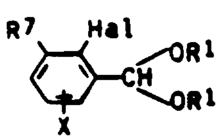

VI

where Hal is halogen, $R^1$ is alkyl of 1 to 6 carbon atoms, $R^7$ is a straight-chain, branched or cyclic hydrocarbon radical of 1 to 18 carbon atoms and X is hydrogen or halogen.

7. The use of a benzaldehyde dialkyl acetal as claimed in claim 5 or 6 for the preparation of a benzaldehyde of the formula

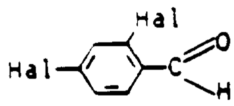

VIII

or

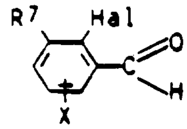

IX

where Hal, $R^7$ and X have the meanings specified in claims 5 and 6.

**Revendications**

1. Procédé de préparation de benzaldéhydedialkylacétals de la formule générale I

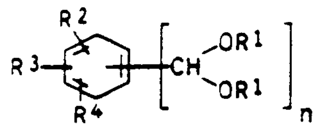

I,

dans laquelle $R^1$ représente un radical alkyle qui comporte de 1 à 6 atomes de carbone, $R^2$, $R^3$ et $R^4$ représentent des atomes d'hydrogène ou d'halogènes, des restes d'hydrocarbures ramifiés ou cycliques, ou des radicaux alcoxy, acyloxy, aryloxy ou aralcoxy et n représente le nombre 1 ou 2, caractérisé en ce que l'on oxyde anodiquement des dérivés benzyliques de la formule générale II

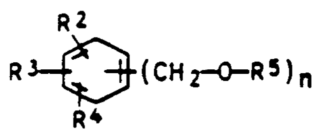

II,

dans laquelle $R^2$, $R^3$, $R^4$ et n possèdent les significations qui leur ont été attribuées ci-dessus, $R^5$ représente un atome d'hydrogène ou un radical $R^6$-CO- et $R^6$ représente un atome d'hydrogène ou un radical alkyle qui comporte de 1 à 6 atomes de carbone, en présence d'un alcool de la formule $R^1$-OH dans laquelle R possède les significations qui lui ont été attribuées ci-dessus.

2. Procédé suivant la revendication 1, caractérisé en ce qu'à titre de dérivés benzyliques de la formule générale II, on utilise des esters benzyliques de la formule générale III

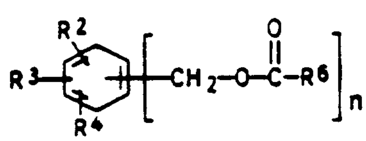

III,

dans laquelle $R^2$, $R^3$, $R^4$, $R^6$ et n ont les significations qui leur ont été attribuées dans la revendication 1, qui ont été obtenus par l'oxydation électrochimique de dérivés du toluène de la formule IV

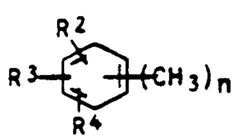

IV,

dans laquelle $R^2$, $R^3$, $R^4$ et n ont les significations qui leur ont été attribuées ci-dessus, en présence d'un acide alcanoïque de la formule $R^6COOH$ dans laquelle $R^6$ possède les significations qui lui ont été attribuées ci-dessus.

**3.** Procédé suivant la revendication 1, caractérisé en ce qu'en vue de l'électro-oxydation, on utilise un électrolyte de la composition suivante : 1 à 49% en poids de dérivé benzylique de la formule II, 50 à 99% en poids d'un alcool de la formule $R^1OH$ et 0,1 à 5% en poids d'un électrolyte auxiliaire.

**4.** Procédé suivant la revendication 1, caractérisé en ce que l'on entreprend l'électro-oxydation à des densités de courant de 0,2 à 25 A/dm$^2$ et à des températures allant jusqu'à 100 °C.

**5.** Benzaldéhydedialkylacétals de la formule générale V

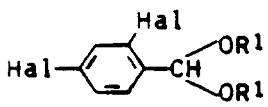

V,

dans laquelle Hal représente un atome d'halogène et $R^1$ représente un radical alkyle qui comporte de 1 à 6 atomes de carbone.

**6.** Benzaldéhydedialkylacétals de la formule générale VI

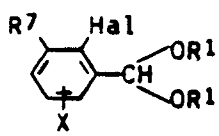

VI,

dans laquelle Hal représente un atome d'halogène, $R^1$ représente un radical alkyle qui comporte de 1 à 6 atomes de carbone, $R^7$ représente un reste d'hydrocarbure à chaîne droite, à chaîne ramifiée ou cyclique, qui comporte de 1 à 18 atomes de carbone et X représente un atome d'hydrogène ou d'halogène.

**7.** Utilisation des benzaldéhydedialkylacétals suivant les revendications 5 et 6 pour la préparation de benzaldéhydes des formules

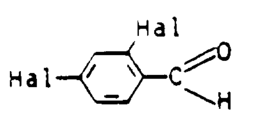

et

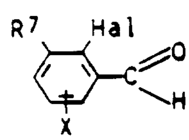

VIII IX

dans lesquelles Hal, $R^7$ et X possèdent les significations qui leur ont été attribuées dans les revendications 5 et 6.